# EUROPEAN PATENT SPECIFICATION

(11) **EP 2 614 066 B1**
(45) Date of publication and mention of the grant of the patent: **29.11.2017**
(21) Application number: 11751912.4
(22) Date of filing: 06.09.2011
(51) Int. Cl.: C07D 501/12, C07D 501/18

(54) **PROCESS FOR THE PRODUCTION OF CEPHALOSPORINS**
VERFAHREN ZUR HERSTELLUNG VON CEPHALOSPORINEN
PROCÉDÉ DE PRODUCTION DE CÉPHALOSPORINES

(30) Priority: 07.09.2010 EP 10175551
(43) Date of publication of application: 17.07.2013
(73) Proprietor: DSM Sinochem Pharmaceuticals Netherlands B.V., 2613 AX Delft (NL)
(72) Inventor: KOSTER, Adrianus Jan, NL-2907 HC Capelle A/d Ijssel (NL); DOES, VAN DER, Thomas, NL-3648 AC Wilnis (NL)
(74) Representative: van Dam, Vincent
(86) International application number: PCT/EP2011/065381
(87) International publication number: WO 2012/032040

(56) References cited:
- EP-A1- 0 532 341
- EP-A1- 1 188 838
- WO-A1-95/04149
- WO-A1-99/50271
- WO-A1-2009/016171
- WO-A2-98/02567

## Description

### Field of the invention

The present invention relates a process for the production of an N-deacylated cephalosporin.

### Background of the invention

The present invention relates to a process for the preparation of cephalosporins and cephalosporin derivatives. More in particular, the present invention relates to the recovery of cephalosporins and derivatives thereof from complex mixtures of cephalosporins and other β-lactam compounds. The invention is also concerned with recovery of deacylated cephalosporins from mixtures of β-lactam compounds and side chains, such as those obtainable by enzymatic side-chain removal.

Semi synthetic routes to prepare cephalosporins mostly start from fermentation products such as penicillin G, penicillin V and cephalosporin C, which are converted to the corresponding β-lactam nuclei, for instance as disclosed in K. Matsumoto, Bioprocess Techn., 16, (1993), 67-88, J.G. Shewale & H. Sivaraman, Process Biochemistry, August 1989, 146-154, T.A. Savidge, Biotechnology of Industrial Antibiotics (Ed. E.J. Vandamme) Marcel Dekker, New York, 1984, or J.G. Shewale et al., Process Biochemistry International, June 1990, 97-103. The obtained β-lactam nuclei are subsequently converted to the desired antibiotic by coupling to a suitable side chain, as has been described in inter alia EP 339751, JP A 53005185 and CH A 640240. By making different combinations of side chains and β-lactam nuclei, a variety of cephalosporin antibiotics may be obtained.

7-Aminodesacetoxycephalosporanic acid (7-ADCA) and 7-aminocephalosporanic acid (7-ACA) are known to be the most important intermediates for the production of cephalosporin antibiotics used in the pharmaceutical industry. 7-ADCA is for example obtained by chemical or enzymatic cleavage (deacylation) of phenylacetyl-desacetoxycephalosporanic acid (PAA-7-ADCA) yielding 7-ADCA and phenylacetic acid (PAA). PAA-7-ADCA is normally produced by chemical treatment of penicillin G sulfoxide, which is formed from penicillin G. In this production process a large amount of chemicals is required to ensure that the desired reaction takes place. This is both expensive and places a heavy burden on waste management. Moreover, the total yield of the process is not very high.

To overcome some of the drawbacks of the chemical process a fermentative process has been disclosed for the production of 7-ADCA, 7-amino-desacetyl cephalosporanic acid (7-ADAC) and 7-ACA, involving fermentative production of N-substituted β-lactams, such as adipyl-7-ADCA, adipyl-7-ADAC or adipyl-7-ACA by a recombinant *Penicillium chrysogenum* strain capable of expressing a gene encoding a desacetoxycephalosporanic acid synthetase (DAOCS) also known as "expandase" from a transgene (EP 532341, EP 540210, WO 93/08287, WO 95/04148). The expandase takes care of the expansion of the 5-membered ring of certain N-acylated penicillanic acids, thereby yielding the corresponding N-acylated desacetoxycephalosporanic acids.

In order to yield the economically most important non-acylated cephalosporins, such as 7-ADCA, 7-ADAC and 7-ACA, the acyl groups are enzymatically removed with a suitable acylase. However, the acyl groups used in these state of the art fermentation processes give rise to a new range of impurities in the end products.

Known processes for recovering chemically or enzymatically produced penicillanic and cephalosporanic acids are not effective for the recovery of the N-substituted β-lactam intermediates and deacylated amino-β-lactams. The main problem with the recovery of the fermentatively produced cephalosporin compounds mentioned above is the complexity of the broth, or culture filtrate. The broth usually comprises various penicillanic acids, such as alpha-aminoadipyl-6-aminopenicillanic acid, alpha-hydroxyadipyl-6-aminopenicillanic acid, 6-aminopenicillanic acid (6-APA), various cephalosporanic acids including alpha-aminoadipyl- and hydroxyadipyl-7-ADCA and a lot of proteinaceous material. Known recovery procedures do not give an acceptable quality of the cephalosporanic acid product in terms of purity. In deacylation this leads to problems in terms of reduced enzyme half-life, slower bioconversion rate and more expenses in the recovery after bioconversion and/or unacceptable contaminant levels. Moreover, after deacylation, such impurities prevent or at least hamper the recovery of the desired deacylated cephalosporin compound of the desired specifications. Finally, it is important to achieve low levels of penicillin impurities since many patients are allergic to penicillins and are treated for that reason with cephalosporin antibiotics.

WO 98/48036 discloses a process for the preparation of cephalosporins having been deacylated at the 7-amino group, by fermentation of a cephalosporin producing microorganism in the presence of a side chain precursor, extraction of the N-substituted cephalosporin compound as present in the fermentation broth or fluid to an organic solvent, back extraction of the N-substituted cephalosporin compound to water, treatment of the aqueous phase with a dicarboxylate acylase and isolation of the crystalline cephalosporin compound according to formula (**1**) from the conversion solution, characterized in that the fermentation broth or fluid is incubated at acidic conditions and an elevated temperature prior to extraction of the N-substituted cephalosporin compound to an organic solvent. WO 99/50271 discloses a similar process but instead of extraction and back-extraction, the N-substituted cephalosporin compound is recovered and purified using chromatography. Although the methods disclosed in WO 98/48036 and WO 99/50271 are very well capable of recovering the N-substituted cephalosporin compound from the broth, fluid or any other complex mixture, they have as a major disadvantage that either organic solvents are used (WO 98/48036) or that a, for industrial purposes, a quite expensive chromatography unit is used (WO 99/50271). Furthermore, both the extraction and chromatography step require pH adjustments (acidification) which consume substantial amounts of acid and, after subsequent neutralization, base with the subsequent formation of salts as a waste stream.

Therefore, there is an urgent need for a novel method which is equally capable of recovering the N-substituted cephalosporin compound from the broth, fluid or any other complex mixture, but which does not have the disadvantages of the prior art processes, such as unacceptable levels of penicillins. It is an object of the present invention to provide such a process.

### Detailed description of the invention

β-Lactamase is defined herein as an enzyme capable of hydrolysing a β-lactam into the corresponding substituted β-amino acid. The β-lactamase is preferably classified according to the Recommendations (1992) of the Nomenclature Committee of the International Union of Biochemistry and Molecular Biology as EC 3.5.2.6. β-Lactamase is also known as penicillinase, penase or cephalosporinase (formerly known as EC 3.5.2.8 but now included in EC 3.5.2.6) and relates to a group of enzymes of varying specificity hydrolysing β-lactams; some act more rapidly on penicillins, some more rapidly on cephalosporins. For the purpose of the invention, a β-lactamase is preferred which acts more on penicillins and less, preferably much less, most preferred not at all, on cephalosporins. By applying such a β-lactamase, the contaminating N-substituted penicillins present in the complex mixture, may selectively by hydrolysed into the corresponding substituted β-amino acids, while leaving the N-substituted cephalosporanic acid unaffected.

β-Lactamases are produced mainly by bacteria and are responsible for their resistance to β-lactam antibiotics. Based on the catalytic mechanism, β-lactamases are divided into several groups (Bush K, Jacoby GA, Medeiros AA.1995. "A functional classification scheme for β-lactamases and its correlation with molecular structure." Antimicrob Agents Chemother. 1995;39: 1211-33) - see Table below.

| **Group** | **Molecular Class** | **Inhibited by clavulanic acid** | **Mechanism** | **Examples** |
|---|---|---|---|---|
| 1 | C | No | Serine-hydrolases | cephalosporinase |
| 2* | A and D | Yes | Serine-hydrolases | penicillinases, cephalosporinases, or both |
| 3 | B | No | Metallo-enzyme | penicillinases, cephalosporinases, carbapenamase |
| 4 | None yet | No | | |

| | | | | |
|---|---|---|---|---|
| * Group 2 is further divided into 2a, 2b, 2c, 2d, 2e and 2f depending on their substrate specificity and molecular class | | | | |

Commercial preparations of β-lactamases are available from various companies. In a preferred embodiment, the β-lactamase from *Escherichia coli* is used.

An effective amount is defined herein as the amount of β-lactamase which is capable of reducing the amount of contaminating N-substituted penicillins present in the complex mixture to a desired residual level. It will be readily understood by the skilled person that the effective amount of β-lactamase depends on the initial concentration of the contaminating N-substituted penicillins present in the complex mixture and the temperature, pH and time at which the β-lactamase reaction may take place. These are conditions which may for instance be dictated by a process for the production of an N-substituted cephalosporin in which the complex mixture as defined herein is one or more of the intermediate stages. The effective amount furthermore depends on the type and source of β-lactamase used.

The invention relates to a process for the production of an N-deacylated cephalosporin of the general formula (**1**) 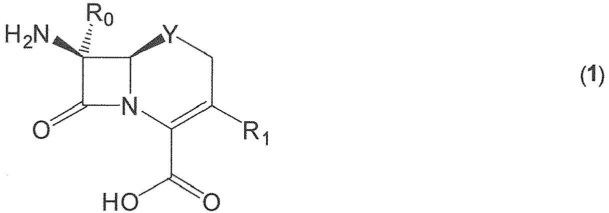 wherein R₀ is hydrogen or C₁₋₃ alkoxy; Y is CH₂, oxygen, sulphur, or an oxidised form of sulphur; R1 is selected from the group consisting of
- hydrogen,
- hydroxy,
- halogen,
- saturated or unsaturated, straight or branched alkyl (1-5 carbon atoms; optionally replaced by one or more heteroatoms), optionally substituted with hydroxy, halogen, aryl, alkoxy (1-3 carbon atoms), or acyl;
- alkoxy (1-3 carbon atoms; optionally replaced by one or more heteroatoms), optionally substituted with hydroxy or halogen; or
- cycloalkyl (3-8 carbon atoms) optionally substituted with hydroxy, halogen, amino;
- aryl;
- heteroaryl,
comprising:
a) fermenting a microorganism capable of producing an N-acylated cephalosporin compound wherein the acyl group is selected from the group consisting of adipyl, succinyl, glutaryl, pimelyl, suberyl, 2-(carboxyethylthio)acetyl, 3-(carboxy-ethylthio)propionyl, higher alkyl saturated and higher alkyl unsaturated dicarboxylic acids and wherein the fermentation broth additionally may comprise N-acylated penicillins and 6-APA; and
b) recovering the N-acylated cephalosporin compound from the fermentation broth obtained in step a) at a pH between 4 and 9; and
c) converting the N-acylated cephalosporin compound obtained in step b) to the corresponding N-deacylated cephalosporin compound in the presence of a deacylating enzyme; and
d) precipitating the N-deacylated cephalosporin compound
characterized in that a β-lactamase is added in step a) and/or step b).

Also described herein is a composition comprising ≥ 85 wt% of an N-deacylated cephalosporin compound and between 0.01 wt% and 2 wt% of an acyl group and ≤200 ppm 6-APA (relative to the N-deacylated cephalosporin), based on the total dry weight of the composition. The N-deacylated cephalosporin has the following general formula (1) 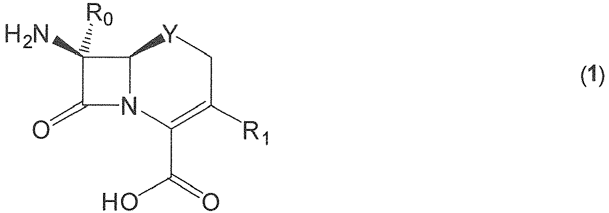 wherein
∘ R₀ is hydrogen or C₁₋₃ alkoxy;
∘ Y is CH₂, oxygen, sulphur, or an oxidised form of sulphur;
∘ R₁ is selected from the group consisting of:
   - hydrogen,
   - hydroxy,
   - halogen,
   - saturated or unsaturated, straight or branched alkyl (1 - 5 carbon atoms; optionally replaced by one or more heteroatoms), optionally substituted with hydroxy, halogen, aryl, alkoxy (1 - 3 carbon atoms), or acyl;
   - alkoxy (1-3 carbon atoms; optionally replaced by one or more heteroatoms), optionally substituted with hydroxy or halogen; or
   - cycloalkyl (3 - 8 carbon atoms) optionally substituted with hydroxy, halogen, amino;
   - aryl;
   - heteroaryl,

Preferred embodiments of the N-deacylated cephalosporin may be selected from the group consisting of 7-ADCA, 7-ACA, 7-amino-3-chloro-3-cephem-4-carboxylic acid (7-ACCA), 7-amino-3-propenyl-3-cephem-4-carboxylic acid 7-PACA and 7-amino-3-carbamoyloxymethyl-3-cephem-4-carboxylic acid (7-ACCCA).

The composition comprises ≥85 wt%, preferably ≥90 wt%, more preferably ≥95 wt% and most preferably ≥98 wt% of the N-deacylated β-lactam compound based on the total dry weight of the composition. Due to the fermentation conditions as described in WO 93/05158 and later documents the resulting fermentation broth contains, sometimes unacceptable high, levels of N-acylated penicillins next to the required N-acylated cephalosporins. Said N-acylated penicillins are converted during enzymatic side chain cleavage to N-deacylated penicillins and the respective side chain. Compositions comprising mainly of cephalosporins and only very small amounts of acyl group in combination with pharmaceutically acceptable low levels of N-deacylated penicillins, i.e. from 10-200 ppm, preferably from 5-100 ppm, more preferably from 2-50 ppm, have hitherto not been disclosed and are highly needed for the production of penicillin-free semi synthetic cephalosporins such as cefadroxil, cephalexin, cephradine and the like. The compositions described hereinmeet these requirements.

The acyl group in the composition originates from the corresponding acylated form of the N-deacylated β-lactam. The acyl group may be any acyl group known from N-acylated β-lactam compounds and are selected from the group consisting of dicarboxylic acids such as adipyl (1,4-dicarboxybutane), succinyl, glutaryl, pimelyl, suberyl, 2-(carboxyethylthio)acetyl, 3-(carboxy-ethylthio)propionyl, higher alkyl saturated and higher alkyl unsaturated dicarboxylic acids. Most preferred is adipyl.

The composition may be in a liquid or solid form. When the composition is in a liquid form, the composition preferably is an aqueous liquid, for instance an aqueous solution of the N-deacylated β-lactam compound. The composition is preferably an aqueous solution comprising the N-deacylated cephalosporin, preferably selected from the group consisting of 7-ADCA, 7-ACA, 7-ACCA, 7-PACA and 7-ACCCA. When the composition is in a solid form, the composition may be a crystalline powder, an agglomerate preferably built up of agglomerated crystals or the composition may be an amorphous powder. Preferably, the composition in solid form comprises only low amounts of water, such as less then 5 wt%, preferably less than 4%, more preferably less than 3%, still more preferably less than 2%, most preferably less than 1%, based on the total weight of the composition.

Most preferred , the composition comprises ≥85 wt% of 7-ADCA. The 7-ADCA comprising composition may be in a liquid form, preferably an aqueous 7-ADCA solution, or may be in a solid form, preferably a crystalline 7-ADCA preparation.

The most preferred acyl-group in the 7-ADCA comprising composition is adipic acid. The composition preferably comprises less than 2 wt% of the adipic acids based on the total dry weight of the composition, more preferably less than 1 wt%, more preferably less than 0,5 wt% and more preferably less than 0,2 wt% and most preferably between 0.05 wt% and 0.2 wt%, next to the low levels of penicillins mentioned above.

Also described herein is a process for the production of the composition as defined hereinbefore and further comprising ≤200 ppm 6-APA based on the total dry weight of the composition, comprising the steps of:
a) fermenting a microorganism capable of producing an N-acylated cephalosporin compound wherein the acyl group is as defined above and wherein the fermentation broth additionally may comprise N-acylated penicillins and 6-APA; and
b) recovering the N-acylated cephalosporin compound from the fermentation broth obtained in step a); and
c) converting the N-acylated cephalosporin compound obtained in step b) to the corresponding N-deacylated cephalosporin compound in the presence of a deacylating enzymes; and
d) precipitating the N-deacylated cephalosporin compound,
characterized in that step b) is carried out a pH between 4 and 9.

Preferably, the fermentation broth is subjected first to biomass separation such as filtration by any suitable means, such as membrane filtration, vacuum filtration, ultrafiltration or a combination thereof, prior to acidification and the optional temperature increase. Any other means of biomass separation is suitable as well.

The main problem with the production and recovery of the fermentatively produced N-acylated cephalosporin compounds mentioned hereinabove is the complexity of the broth, or culture filtrate. The broth usually comprises various penicillanic acids, such as 6-APA, α-amino-adipyl-6-APA, adipyl-6-APA, α-hydroxyadipyl-6-APA, and various cephalosporanic acids including α-aminoadipyl-7-ADCA and α-hydroxyadipyl-7-ADCA and a lot of proteinaceous material. We have now surprisingly found that the amount of contaminating penicillanic acids can be reduced significantly by using an effective amount of a β-lactamase. Therefore, in the process of the invention an effective amount of a β-lactamase (as defined hereinbefore) is added in step a) or step b) or in both step a) and step b). The advantage of adding a β-lactamase is that contaminating compounds which may be formed during the fermentation step a) or during the recovery step b) are degraded by β-lactamase. For example, adipyl-6-APA may be formed during fermentation processes for the production of adipyl-7-ADCA. Adding β-lactamase results in a significant and substantial degradation of the adipyl-6-APA. The advantage is that in the subsequent enzymatic deacylation step leading to 7-ADCA, much less 6-APA is formed which results in 7-ADCA of much higher purity. This eventually also leads to semi synthetic cephalosporins with substantial reduced levels of the corresponding semi synthetic penicillins.

In a further preferred embodiment of the process of the invention, in addition to adding β-lactamase as described hereinbefore, also an effective amount of carbon dioxide may be added during step b); or between step c) and step d); or during step b) and between step c) and step d), essentially as disclosed in WO98/48036. Carbon dioxide may be added in any suitable way, such as in a solid or gaseous form or as a solution of carbonate ions. The reaction with the carbon dioxide is typically carried out at pH 5-7 and at a temperature between 10-60°C, preferably 20-40°C, under conditions of saturated molecular carbon dioxide concentrations during 0.5-10 hours. When added in gaseous form, the overpressure of CO₂ preferably is between 0 and 10 bar.

Also described herein is a process for the production of a semi synthetic cephalosporin comprising contacting the N-deacylated cephalosporin present in the abovementioned composition and produced by the process of the invention with an activated side chain in the presence of an enzyme. Preferably, the N-deacylated cephalosporin is 7-ADCA and the semi synthetic cephalosporin is selected from the group consisting of cephalexin, cefadroxil and cephradine which may be prepared chemically or enzymatically according to procedures known to the skilled person. Surprisingly, the semi synthetic cephalosporins so obtained contain pharmaceutically acceptable low levels, i.e. from 2-10 ppm, of the corresponding semi synthetic penicillin derivatives. Examples are cefadroxil comprising from 2-10 ppm of the corresponding semi synthetic penicillin amoxicillin or cephalexin comprising from 2-10 ppm of the corresponding semi synthetic penicillin ampicillin or cephradine comprising from 2-10 ppm of N-dihydrophenylglycine-6-aminopenicillanic acid.

### MATERIALS AND METHODS

Difco™ Penase concentrate (10,000,000 IU/ml) was purchased from Becton Dickinson and Company BD Diagnostic Systems, PO Box 999, Sparks MD 21152-0999 US BD, catalogue nr 234610.

### EXAMPLES

### Example 1

### Production of 7-ADCA from adipyl-7-ADCA

A broth containing adipyl-7-ADCA was obtained by fermentation of a *Penicillium chrysogenum strain* transformed with a gene encoding expandase as described in WO 93/05158. The mycelium was removed by microfiltration. 180 ml of Difco™ Penase concentrate was added to 7 L of a microfiltrate containing adipyl-7-ADCA. The mixture was incubated at pH 5.8 for 120 minutes at a temperature of 35°C (run 7) and CO₂ was added during 2 hours as a gas at a pressure of 5 bar. Two different routes were investigated:

### Route 1:

1. Degradation of adipyl-6-aminopenicllanic acid by Penase for 2 hours at pH 5.8 and 35°C
2. Ultrafiltration of samples (10 kD) taken after degradation
3. Removal of by-products from permeate by pH increase and filtration over a Z2000 Seitz filter
4. Enzymatic conversion of adipyl-7-ADCA to 7-ADCA using immobilised penicillin acylase
5. Acidification
6. Ultrafiltration of the 7-ADCA-solution
7. CO₂-treatment for 2 hours at 5 bar
8. Concentration by evaporation at 40°C
9. Isolation of 7-ADCA by means of crystallisation

### Route 2:

1. Degradation of adipyl-6-aminopenicllanic acid by Penase for 2 hours at pH 5.8 and 35°C
2. Ultrafiltration of samples (10 kD) taken after degradation
3. CO₂-treatment for 2 hours at 5 bar
4. Removal of by-products from permeate by pH increase and filtration over a Z2000 Seitz filter
5. Enzymatic conversion of adipyl-7-ADCA to 7-ADCA using immobilised penicillin acylase
6. Acidification
7. Concentration by evaporation at 40°C
8. Isolation of 7-ADCA by means of crystallisation

**Table 1 Reduction of 6-APA in 7-ADCA samples**

| **Route** | **Penase treatment** | **CO₂ treatment** | **Reduction of 6-APA (%)** | **Run** |
|---|---|---|---|---|
| 1 | No | No | 67.4 | 3 |
| 1 | Yes | No | 85.8 | 6 |
| 1 | Yes | Yes | 98.7 | 7 |
| 2 | Yes | No | 95.0 | 7 |
| 2 | Yes | Yes | 99.1 | 7 |

**Table 2 Analysis of crystal samples**

| | **Run 3** | **Run 6** | **Run 7 Route 1** |
|---|---|---|---|
| 7-ADCA | 90.5% | 88.5% | 95.1% |
| Adipyl-7-ADCA | 0.1% | <0.1% | 0.2% |
| Δ²-7-ADCA | <0.1% | <0.1% | <0.1% |
| Unknown | 0.3% | 0.5% | 0.2% |
| Hydroxyadipyl-7-ADCA | 0.1% | <0.1% | <0.1% |
| Total cephalosporin impurities | 1.3% | 1.3% | 1.1% |
| Adipic acid | 5,509 ppm | 10,421 ppm | 4,340 ppm |
| 6-APA | 26,000 ppm | 7,300 ppm | 760 ppm |

**Table 3 Analysis of crystal samples**

| | **Run 7, route 1** | **Run 7, route 2** |
|---|---|---|
| 7-ADCA | 95.4% | 95.8% |
| Adipyl-7-ADCA | 0.2% | 0.2% |
| Δ²-7-ADCA | <0.1% | <0.1% |
| Unknown | 0.2% | 0.2% |
| Hydroxyadipyl-7-ADCA | <0.1% | <0.1% |
| Total cephalosporin impurities | 1.1% | 0.8% |
| 6-APA | 800 ppm | 550 ppm |

### Example 2

### Quality of cephalexin produced from the crude 7-ADCA sample

A reactor was loaded with 7-ADCA (6.50 g; run 7, route 1; as obtained in Example 1) and water (40.4 ml). The temperature was adjusted to 20°C and ammonia (25%) was added to reach pH 7.2. Then immobilized penicillin acylase (8.00 g; the *Escherichia coli* wild type penicillin G acylase with mutations B:F24A and B:V148L as disclosed in Example 1 of WO 2010/072765 immobilized according to the method disclosed in EP 839192 and EP 222462) was added and a solution of D-phenylglycine methyl ester methylsulphonic acid (8.00 g) in water (5.0 g) was dosed in 180 minutes and the pH was kept to 7.2. Cephalexin seed crystals (0.25 g) were added and after 240 minutes the pH was decreased to 6.0 with sulphuric acid 25%. Cephalexin was isolated using a sieve as described in WO 98/56486. The wet cake and mother liquors were combined and cooled to 5°C and sulphuric acid 25% (6.8 g) and charcoal (0.5 g) were added to give a pH of 1.50. After stirring for 5 min, the suspension was filtrated through a Seitz filter and washed with water. This solution is dosed to a reactor filled with water (20.0 ml) and cephalexin crystals (0.5 g) at 30°C and pH 5.0 using ammonia 25%. The crystals were collected by filtration and washed with water and acetone and dried at 45°C. The amount of ampicillin present in the cephalexin crystals was <10 ppm (whereas with a normal cephalexin synthesis the ampicillin content was 17 ppm).

## Claims

1. A process for the production of an N-deacylated cephalosporin of the general formula (**1**) 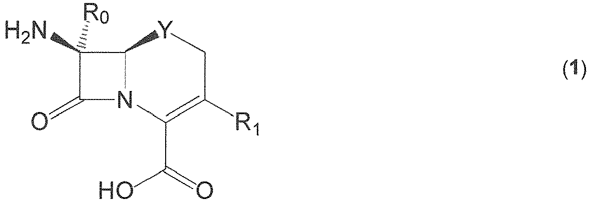 wherein R₀ is hydrogen or C₁₋₃ alkoxy; Y is CH₂, oxygen, sulphur, or an oxidised form of sulphur; R₁ is selected from the group consisting of
• hydrogen,
• hydroxy,
• halogen,
• saturated or unsaturated, straight or branched alkyl (1-5 carbon atoms; optionally replaced by one or more heteroatoms), optionally substituted with hydroxy, halogen, aryl, alkoxy (1-3 carbon atoms), or acyl;
• alkoxy (1-3 carbon atoms; optionally replaced by one or more heteroatoms), optionally substituted with hydroxy or halogen; or
• cycloalkyl (3-8 carbon atoms) optionally substituted with hydroxy, halogen, amino;
• aryl;
• heteroaryl,
comprising:
a) fermenting a microorganism capable of producing an N-acylated cephalosporin compound wherein the acyl group is selected from the group consisting of adipyl, succinyl, glutaryl, pimelyl, suberyl, 2-(carboxyethylthio)acetyl, 3-(carboxy-ethylthio)propionyl, higher alkyl saturated and higher alkyl unsaturated dicarboxylic acids and wherein the fermentation broth additionally may comprise N-acylated penicillins and 6-APA; and
b) recovering the N-acylated cephalosporin compound from the fermentation broth obtained in step a) at a pH between 4 and 9; and
c) converting the N-acylated cephalosporin compound obtained in step b) to the corresponding N-deacylated cephalosporin compound in the presence of a deacylating enzyme; and
d) precipitating the N-deacylated cephalosporin compound **characterized in that** a β-lactamase is added in step a) and/or step b).

2. Process according to claim 1 wherein R₀ is hydrogen, Y is sulphur and R₁ is methyl.

3. Process according to any one of claims 1 to 2 wherein said N-acylated cephalosporin compound is an N-adipyl cephalosporin.

4. A process according to any one of claims 1 to 3 wherein CO₂ is added
• during step b); or
• between step c) and step d); or
• during step b) and between step c) and step d)

## Patentansprüche

1. Verfahren zur Herstellung eines N-deacylierten Cephalosporins der allgemeinen Formel (1) 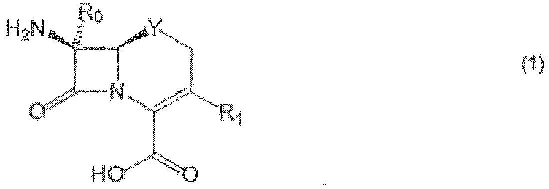 wobei R₀ Wasserstoff oder C₁₋₃-Alkoxy ist, Y = CH₂, Sauerstoff, Schwefel oder eine oxidierte Form von Schwefel ist, R₁ aus der Gruppe ausgewählt ist, die aus Folgenden besteht:
• Wasserstoff;
• Hydroxy;
• Halogen;
• gesättigtes oder ungesättigtes, geradkettiges oder verzweigtes Alkyl (1-5 Kohlenstoffatome; gegebenenfalls ersetzt durch ein oder mehrere Heteroatome), gegebenenfalls substituiert mit Hydroxy, Halogen, Aryl, Alkoxy (1-3 Kohlenstoffatome) oder Acyl;
• Alkoxy (1-3 Kohlenstoffatome; gegebenenfalls ersetzt durch ein oder mehrere Heteroatome), gegebenenfalls substituiert mit Hydroxy oder Halogen; oder
• Cycloalkyl (3-8 Kohlenstoffatome), gegebenenfalls substituiert mit Hydroxy, Halogen, Amino;
• Aryl;
• Heteroaryl;
umfassend:
a) Fermentieren eines Mikroorganismus, der eine N-acylierte Cephalosporinverbindung produzieren kann, wobei die Acylgruppe aus der Gruppe ausgewählt ist, die aus Adipyl, Succinyl, Glutaryl, Pimelyl, Suberyl, 2-(Carboxyethylthio)acetyl, 3-(Carboxyethylthio)propionyl, Höher-Alkyl-gesättigten und Höher-Alkyl-ungesättigten Dicarbonsäuren besteht, und wobei die Fermentationsbrühe zusätzlich N-acylierte Penicilline und 6-APA umfassen kann; und
b) Gewinnen der N-acylierten Cephalosporinverbindung aus der in Schritt a) erhaltenen Fermentationsbrühe bei einem pH-Wert zwischen 4 und 9; und
c) Umwandeln der in Schritt b) erhaltenen N-acylierten Cephalosporinverbindung in die entsprechende N-deacylierte Cephalosporinverbindung in Gegenwart eines deacylierenden Enzyms; und
d) Ausfällen der N-deacylierten Cephalosporinverbindung;
**dadurch gekennzeichnet, dass** in Schritt a) und/oder Schritt b) eine β-Lactamase hinzugefügt wird.

2. Verfahren gemäß Anspruch 1, wobei R₀ Wasserstoff ist, Y Schwefel ist und R₁ Methyl ist.

3. Verfahren gemäß einem der Ansprüche 1 bis 2, wobei die N-acylierte Cephalosporinverbindung ein N-Adipylcephalosporin ist.

4. Verfahren gemäß einem der Ansprüche 1 bis 3, wobei CO₂ hinzugefügt wird:
• während Schritt b); oder
• zwischen Schritt c) und Schritt d); oder
• während Schritt b) und zwischen Schritt c) und Schritt d).

## Revendications

1. Procédé de production d'une céphalosporine N-désacylée de formule générale (1) 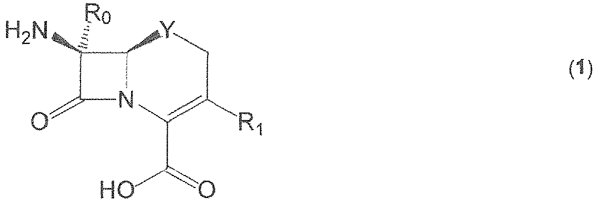 dans laquelle R₀ représente un atome d'hydrogène ou un groupe C₁₋₃ alkoxy; Y représente CH₂, un atome d'oxygène, de soufre, ou une forme oxydée de soufre ; R₁ est choisi dans le groupe constitué de
• un atome d'hydrogène,
• un groupe hydroxy,
• un halogène,
• un groupe alkyle linéaire ou ramifié, saturé ou insaturé (1 à 5 atomes de carbone ; éventuellement remplacés par un ou plusieurs hétéroatomes), éventuellement substitué par un groupe hydroxy, halogène, aryle, alkoxy (1 à 3 atomes de carbone), ou acyle ;
• un groupe alkoxy (1 à 3 atomes de carbone ; éventuellement remplacés par un ou plusieurs hétéroatomes), éventuellement substitué par un groupe hydroxy ou halogène ; ou
• un groupe cycloalkyle (3 à 8 atomes de carbone) éventuellement substitué par un groupe hydroxy, halogène, amino ;
• un groupe aryle ;
• un groupe hétéroaryle,
comprenant :
a) la fermentation d'un micro-organisme capable de produire un composé de céphalosporine N-acylée dans lequel le groupe acyle est choisi dans le groupe constitué des groupes adipyle, succinyle, glutaryle, pimélyle, subéryle, 2-(carboxyéthylthio)acétyle,3-(carboxyéthylthio)propionyle, acides dicarboxyliques saturés d'alkyle supérieur et insaturés d'alkyle supérieur et dans laquelle le bouillon de fermentation peut comprendre en outre des pénicillines N-acylées et du 6-APA ; et
b) la récupération du composé de céphalosporine N-acylée à partir du bouillon de fermentation obtenu dans l'étape a) à un pH situé entre 4 et 9 ; et
c) la conversion du composé de céphalosporine N-acylée obtenu dans l'étape b) en le composé de céphalosporine N-désacylée correspondant en présence d'une enzyme de désacylation ; et
d) la précipitation du composé de céphalosporine N-désacylée
**caractérisé en ce qu'**une β-lactamase est ajoutée à l'étape a) et/ou l'étape b).

2. Procédé selon la revendication 1, dans lequel R₀ représente un atome d'hydrogène, Y représente un atome de soufre et R₁ représente un groupe méthyle.

3. Procédé selon l'une quelconque des revendications 1 ou 2, dans lequel ledit composé de céphalosporine N-acylée est une N-adipyl-céphalosporine.

4. Procédé selon l'une quelconque des revendications 1 à 3, dans lequel du CO₂ est ajouté
• durant l'étape b) ; ou
• entre l'étape c) et l'étape d) ; ou
• durant l'étape b) et entre l'étape c) et l'étape d).
